# EUROPEAN PATENT APPLICATION

(11) **EP 3 093 344 A1**
(43) Date of publication of application: **16.11.2016**
(21) Application number: 14877857.4
(22) Date of filing: 29.12.2014
(51) Int. Cl.: C12N 15/55, C12N 9/82, A61K 38/50, A61P 35/02

(54) **RECOMBINANT L-ASPARAGINASE FROM ZYMOMONAS**

(30) Priority: 10.01.2014 BR 14000585
(71) Applicant: Instituto Alberto Luiz Coimbra De Pós Graduação E Pesquisa De Engenharia-COPPE/UFRJ, 21949-900 Rio de Janeiro - RJ (BR)
(72) Inventor: MOITINHO ALVES, Tito Lívio, 21940-006 Rio de Janeiro - RJ (BR); EISNFELDT, Karen, 21921-550 Rio de Janeiro - RJ (BR); CAVALCANTE BAPTISTA, Isis, 21230-700 Rio de Janeiro - RJ (BR); VOLCAN ALMEIDA, Rodrigo, 21941-070 Rio de Janeiro - RJ (BR); SOBRAL DA COSTA, Elaine, 22261-050 Rio de Janeiro - RJ (BR); MENKS RIBEIRO, Maria Cecília, 20240-240 Rio de Janeiro - RJ (BR); POIROT LAND, Marcelo Greradin, 24240-003 Rio de Janeiro - RJ (BR); LARENTIS, Ariane Leites, 22231-130 Rio de Janeiro - RJ (BR)
(74) Representative: Studio Torta S.p.A.
(86) International application number: PCT/BR2014/000455
(87) International publication number: WO 2015/103681

(57) **Abstract**

The present invention relates to the construction and optimization of synthetic genes from the *Zymomonas mobilis* L-asparaginase gene, the method for cloning same and expression thereof in *Escherichia coli.* The purpose of the production of said enzyme is for producing high levels of a novel L-asparaginase that can be used in L-asparaginase-based pharmaceutical compositions for treating cancer, tumours and diseases involving cell proliferation, as well as for other medical applications.

## Description

### FIELD OF THE INVENTION

The present invention refers to the construction and optimization of synthetic L-asparaginase gene *Zymomonas sp.,* preferably *Zymomonas mobilis* and the construction of plasmids for expression of intracellular and extracellular enzyme in bacteria and yeast. The invention relates to the production of this enzyme in submerged cultures, for use in pharmaceutical preparations L-asparaginase base used in the treatment of cancer, tumors, diseases of cell proliferation, as well as other medical applications.

### BACKGROUND OF INVENTION

For the treatment of a variety of lymphoproliferative disorders and lymphomas, particularly for acute lymphoblastic leukemia (ALL), the enzyme L-asparaginase (L-asparagine amino hydrolase, EC 3.5.1.1) is used as the chemotherapeutic agent (Narta et al. Critical Reviews in Oncology / Hematology 61, 208-221, 2007;. Verma et al Critical Reviews in Biotechnology 27, 45-62, 2007). The antineoplastic activity of L-asparaginase occurs causes the depletion of exogenous supply of L-asparagine for the cells, since the malignant cells synthesize L-asparagine slowly in comparison with its need and rely on an exogenous supply of this amino acid. On the other hand, normal cells can normally synthesize the amino acid, and therefore are not damaged by the use of L-asparaginase (Lee et al Applied Biochemistry and Biotechnology 22 (1): 1-11, 1989; Duval et al Blood. 99 (8), 2734-2739, 2002; Kotzia Labrou and Journal of Biotechnology 119, 309-323, 2005 Rizzari et al Current Opinion in Oncology 25, S1-S9, 2013).

On the market are available formulations of native enzyme obtained from the microorganisms *Escherichia coli and Erwinia chrysanthemi* L-asparaginase of *E.coli* conjugated to polyethylene glycol (PEG) (Kurtzberg. In: Holland-Frei Cancer Medicine, 5th Ed., Hamilton Publisher (ON): BC Decker, 2000 Van Den Berg Leukemia & Lymphoma, 52 (2), 168-178, 2011). During the treatment, the main problem in the use of L-asparaginase is the clinical hypersensitivity developed by patients treated with the unmodified forms of the enzyme. Studies with L-asparaginases from *E. coli* and *Erwinia chrysanthemi* showed that both enzymes have high immunogenicity (Narta et al. Critical s Revie in Oncology / Hematology. 61, 208-221, 2007). However, these enzymes (from *E. coli* and *Erwinia chrysanthemi*) serve as an alternative if the patient develops sensitivity to one of them ( Kotzia and Labrou. Journal of Biotechnology. 119, 309-323, 2005 Rizzari et al. Current Opinion in Oncology. 25, S1-S9, 2013). Another alternative to the problem of hypersensitivity is to use another type of L-asparaginase (obtained from a different micro-organism or conjugated to polyethylene glycol), therefore it is very important to search for new sources of L-asparaginase which have antileukemic activity. A new source of L-asparaginase enzyme is the *Zymomonas mobilis* bacteria.

The *Zymomonas mobilis* bacterium is reported to have therapeutic properties. There are reports of therapeutic applications of *Zymomonas* cultures in cases of bacterial enterocolitis and gynecological infections (Wanik and Silva Antibiotics Institute Journal 11, 69-71, 1971; Lopes et al Journal of Antibiotics Institute 20, 69- 77, 1980).

However, low productivity of L-asparaginase enzyme by *Z. mobilis* becomes an obstacle to the study and production of the enzyme from this organism. This obstacle can be overcome by the production of the recombinant enzyme at high concentrations, using genetic engineering.

To meet the industrial demand, proteins of interest can be produced in large quantities using genetic engineering (Demain and Vaishnav. Biotechnology Advances. 27, 297-306, 2009). The bacterium *Escherichia coli* is the most widely used expression systems for recombinant proteins, including on a commercial scale (Baneyx Current Opinion in Biotechnology 10, 411-421, 1999; TERPE Applied Microbiology and Biotechnology 72, 211-222., 2006.) This is because, besides offering several advantages such as the ability to grow rapidly at high cell concentrations and cheap substrates, it has well characterized genetic and there are several vectors and commercially available mutant strains (Baneyx. Current Opinion in Biotechnology. 10, 411 -421, 1999; Page and PETI Protein Expression and Purification 51: 1-10, 2007). In addition, cells from *E.coli* have ability to accumulate over 80% of its dry weight in recombinant protein (Demain and Vaishnav. Biotechnology Advances. 27, 297-306, 2009).

### PRIOR ART

The gene manipulation technique has been widely used to develop production systems of the different types of proteins with diverse purposes. The book Molecular Cloning: A Laboratory Manual (Sambrook J, Fritsch EF, Maniatis T. 1989. Molecular Cloning: A Laboratory Manual, 2nd edition Cold Spring Harbor Laboratory Press, New York) describes in detail the process of genetic manipulation for obtaining recombinant cells.

The bacterium *Escherichia coli* is the most widely used expression systems for recombinant proteins, including on a commercial scale (Baneyx Current Opinion in Biotechnology 10, 411 -421, 1999; TERPE Applied Microbiology and Biotechnology 72, 211-222., 2006.). Several patent documents use *E. coli* as the micro-organism for expression of recombinant proteins, including L-asparaginases. As well as some patent documents produce recombinant L-asparaginases from different microorganisms in hosts such as *Escherichia coli* and yeasts. However, there are no documents reporting the cloning and expression of recombinant L-asparaginase *Zymomonas sp*. in any micro-organism.

In the patent document WO2003/0186380 are described recombinant methods for producing a polypeptide with at least 70% identity with the sequence of amino acids L-asparaginase ATCC6051A of *Bacillus subtilis*

There are documents that report inventions of modified forms of L-asparaginase. The invention US2012/0100121 refers to a chemically modified form of the enzyme from *Erwinia* L-asparaginase, conjugated with a molecule of polyethylene glycol, particularly with molecular weight less than or equal to 5000 Da, and its clinical use. The document US20100284982 describes compositions for the transporting of L-asparaginase through the cell membrane of erythrocytes, the preparation process of these compositions and the method of its clinical use.

In the patent document WO2008/011234A2 it is described the cloning and production of L-asparaginase II from *Escherichia coli* using *Escherichia coli* as a host, resulting in a recombinant strain of *E. coli* with a vector extrachromosomal and chromosomal DNA coding for the same L - asparaginase II. The document WO2010/015264A1 relates to cloning, production and characterization of the L-asparaginase enzyme of *Helicobacter pylori* CCUG 17874 using *Escherichia coli* as a host. As for the Brazilian patent application PI0406168-3 it relates to production of L-asparaginase *Saccharomyces cerevisiae* by cloning the gene encoding such enzyme in a methylotrophic yeast. The patent application PI 0404952-7, which has the same applicant of the present one, refers to the obtaining process of the native L-asparaginase enzyme of *Zymomonas mobilis,* by culture of wild *Zymomonas mobilis* for production of the enzyme, in which activity was obtained 37.79 IU/g in 33 hours of cell culture.

### SUMMARY OF THE INVENTION

The present invention reports the construction and optimization of synthetic L-asparaginase of *Zymomonas mobilis* gene as well as the construction of plasmids for expression of intracellular and extracellular enzyme in *Escherichia coli,* the subsequent insertion of these plasmids into *Escherichia coli* and the production of enzyme in submerged fermentation. The development of this recombinant enzyme aim for its use in pharmaceutical compositions intended for use in medical applications, such as in the treatment of cancer, tumors and diseases in which cell proliferation is involved, providing an alternative to existing drugs.

The products developed in this invention represent a new way to treat cancer, tumors and diseases in which cell proliferation is involved. In addition, the developed techniques provide a high production output and productivity, which is reflected in lower cost of the final product.

### BRIEF DESCRIPTION OF THE FIGURES

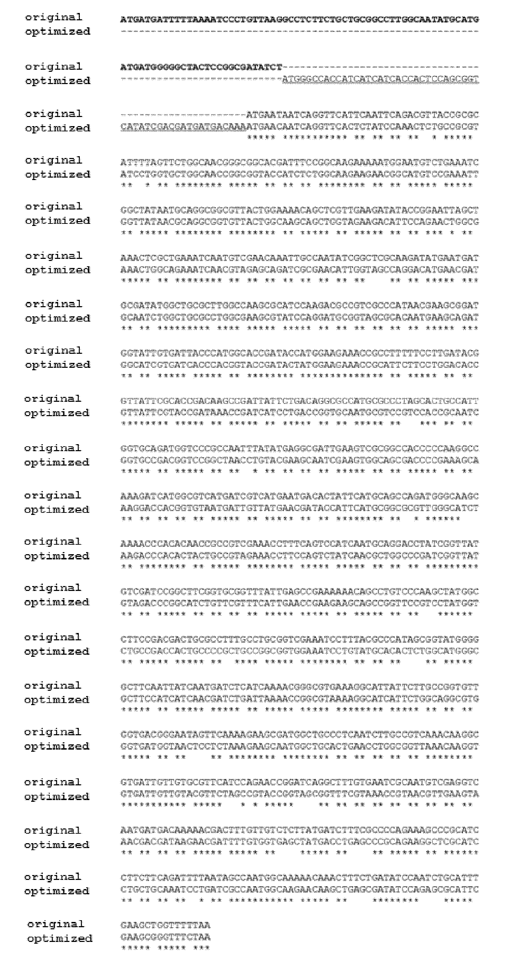

Figure 1 shows the alignment using the CLUSTAL 2.0.1 Multiple Sequence Alignment software of the 1-asparaginase gene (original) with L-asparaginase gene (optimized). The underlined nucleotides in bold represent those that are part of the signal peptide of the original gene that were withdrawn from the optimized gene. The underlined nucleotides reffers to the histidine tail and enterokinase cleavage site added to the optimized gene.

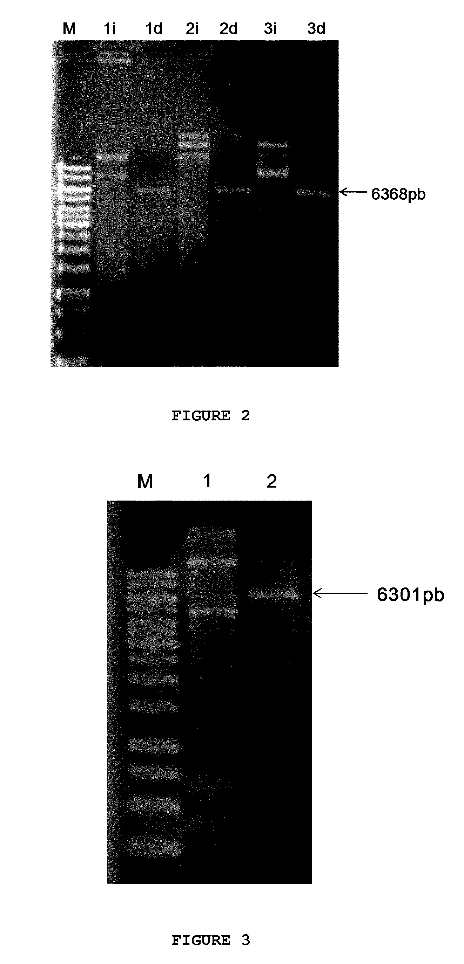

Figure 2 represents the electrophoretic pattern and digestion of plasmids extracted from three clones of *Escherichia coli* BL21(DE3)/pET26b/asparaginase, digested with the restriction enzyme (Xho\). M Marker 1kb GeneRuler™ DNA Ladder (Fermentas), i-vector pET26b/intact asparaginase, d-vector pET26b/asparaginase digested with the Xhol enzyme. The arrow indicates the 6368 bp fragment corresponding to the linearized pET26b/asparaginase vector.
Figure 3 represents the electrophoretic pattern and digesting of the plasmids extracted of 3 clones of *Escherichia coli* BL21 (DE3)/pET28a/asparaginase, digested with the restriction enzyme (Xho\). M GeneRuler™ DNA Marker 1kb Ladder (Fermentas), 1-intact pET28a/asparaginase vector, 2- pET28a/asparaginase vector digested with the enzyme Xhol. The arrow indicates the 6301 bp fragment corresponding to the linearized pET28a/asparaginase vector.

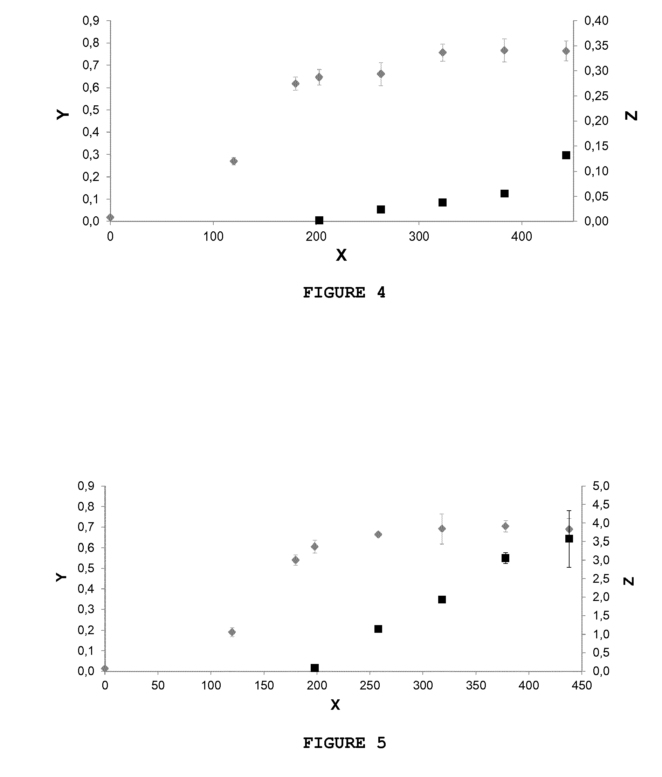

Figure 4 shows the production chart of extracellular L-asparaginase by the *Escherichia coli* BL21(DE3)/pET26b/asparaginase bacteria and cell growth in the bioreactor using LB medium. The Y-axis represents the cell concentration (g/L). The Z axis represents the enzyme activity (IU/ml). The X axis represents time (minutes). ■ - Enzyme Activity (IU/ml); ◆ - Cell Concentration (g/L). The bars represent standard deviations.
Figure 5 shows the production chart of intracellular L-asparaginase by the *Escherichia coli* BL21 (DE3)/pET28a/asparaginase bacteria and cell growth in the bioreactor using LB medium. The Y-axis represents the cell concentration (g/L). The Z axis represents the enzyme activity (IU/ml). The X axis represents time (minutes). ■ - Enzyme Activity (IU/ml); ◆ - Cell Concentration (g/L). The bars represent standard deviations.

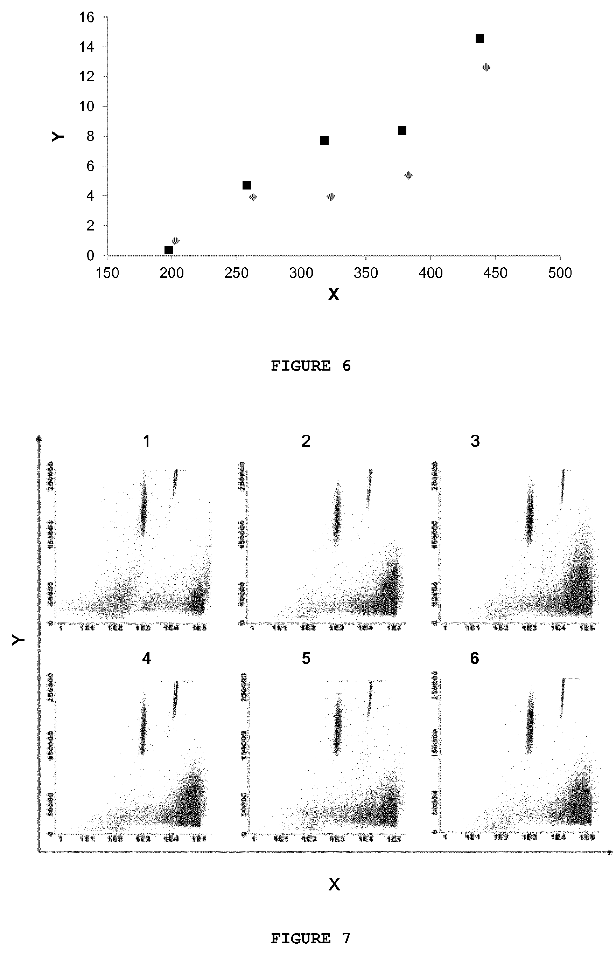

Figure 6 shows the specific activity graphic of intracellular L-asparaginase (using *Escherichia coli* BL21(DE3)/pET28a/asparaginase) and extracellular (using *Escherichia coli* BL21(DE3)/pET26b/asparaginase) produced in the bioreactor using LB medium. The Y-axis represents the specific activity of the L-asparaginase (IU/mg). The X axis represents time (minutes). ■ - intracellular L-asparaginase (IU/mg); ◆ - extracellular L-asparaginase (IU/mg).
Figure 7 shows the cell viability analysis chart by flow cytometry, through staining with propidium iodide (PI) in primary sample of bone marrow exposed to various concentrations of L-asparaginase recombinant of Z mobilis. The Y-axis represents the lateral spreading of the cells. The X-axis represents the fluorescence intensity per IP. 1- control, 2- 1.0 IU/mL, 3- 0.5 IU/mL, 4-0,1UI/mL, 5- 0,05UI/ml 0.025, 6- IU/ml.

### DETAILED DESCRIPTION OF THE INVENTION

Two vectors were constructed containing the gene that encodes the enzyme L-asparaginase from *Zymomonas mobilis,* a vector for the intracellular enzyme expression and another vector for the extracellular enzyme expression. In both vectors the gene encoding L-asparaginase enzyme was based on the sequence of *Zymomonas mobilis subsp. mobilis* ZM4 deposited in genbank database with 3,189,240 ID reference to the gene and YP_163418.1 to the amino acid sequence, which can be accessed at http://www.ncbt.nlm.nih.gov address. By inserting these vectors in *Escherichia coli* were obtained two distinct clones, both able to express the recombinant L-asparaginase protein.

The invention will be detailed by examples in the following text. The invention is not limited by the examples presented below, those being for illustrative purposes, for a better understanding of the invention reported in this document.

### Example 1 - Synthesis of the 1-asparaginase gene

The gene type II of *Zymomonas mobilis* of the L-asparaginase gene was chemically synthesized by the company Epoch Life Science Inc. The design of the gene was performed using the sequence of the 1 01 nucleotides of the strain *Zymomonas mobilis subsp. mobilis* ZM4 gene deposited in the GenBank data base by the 3189240 ID reference and illustrated herein as SEQ ID NO: 2, it sequence encodes a protein of 366 amino acids. The gene was synthesized based on the sequence of the L-asparaginase gene of *Zymomonas mobilis subsp. mobilis* ZM4 noted above, removing the nucleotides encoding the first 29 amino acids of the sequence, as those are part of a signal sequence (signal peptide). To that sequence was added a nucleotide sequence encoding six histidine, allowing the expression of the fused protein with a histidine tag at its N-terminus far end. Were also added nucleotides encoding the amino acid sequence: Asp-Asp-Asp-Asp-Lys, a cleavage site of the enterokinase enzyme for later removal of the histidine tag. The codons used in the synthetic gene were optimized by the most frequent codons used by *Escherichia coli.* The synthetic gene, flanked by restriction enzymes Ncol and Xhol, was inserted in the pET26b vector (Novagen), which has a signal sequence (signal peptide), pelB (Erwinia carotovora native), to export the protein to the space periplasmic, where it will be secreted into the culture medium. This same synthetic gene, flanked by the restriction enzymes Nco\ and Xho\, was also inserted into pET28a vector (Novagen) to express the protein in the cytoplasm. The sequence of the optimized and synthesized gene is shown in SEQ ID NO: 1. The sequence of the pET26b plasmid containing the optimized gene is shown in SEQ ID NO: 3 and the sequence of the pET28a plasmid containing the optimized gene is shown in SEQ ID NO: 4. The optimized sequence SEQ ID NO: 1, has a translation represented in SEQ ID NO: 5.

The alignment of the original L-asparaginase gene (SEQ ID NO: 2) with L-asparaginase optimized gene (SEQ ID NO: 1) is shown in Figure 1.

### Example 2 - Transformation into Escherichia coli

Eletrocompetent *E. coli* DH5a and *E. coli* BL21 (DE3) cells were used as hosts for the plasmids pET26b / asparaginase and pET28a / asparaginase. The insertion of those plasmids in the cells was performed by electroporation. For the electroporation, the plasmids and 100 ul of eletrocompetent *E. coli* were gently mixed to avoid the formation of bubbles. Each mixture was transferred to a cuvette of 0.2 cm thick between the electrodes, and then subjected to an electric discharge for about 5 ms, with a voltage of 2.5 kV, capacitance of 25 pF and resistance of 200 Q at a eletroporator Gene Pulser® II (Bio-Rad). After the electric shock, were quickly added 1 mL of sterile LB medium and the mixture was incubated at 37 ° C under agitation at 200 rpm for 1 hour. After this period, 200pL of the mixture were spread on LB agar plate containing 50 pg / ml of canamictna. The plates were incubated at 37 ° C for 16 hours. This procedure was first carried out with DH5et *E. coli* strain and after confirmation of the clones, the plasmids were extracted and transformed into the expression strain *E. coli* BL21 (DE3).

The selection of clones transformed with the plasmids was made by spreading the cells on LB agar with selective pressure (use of the kanamycin antibiotic). To make the selection, some colonies present on the plate were selected, and each inoculated into 10 mL of liquid LB medium (5 g/L yeast extract, 10 g/L of tryptone, 10 g/L of NaCl) with 50 pg/ml of kanamycin and 1% of glucose and incubated at 37 ° C and 200 rpm for 16 hours. From these crops were made glycerol stocks (called Mother Stock), stored at -8Q ° C, and the plasmid extractions for the confirmation of clones (electrophoretic and digestion default). The electrophoretic and digestion pattern of the clones of BL21

(DE3)/pET26b/asparaginase *Escherichia coli* and BL21 (DE3)/pET28a/asparaginase *Escherichia coli* are shown in Figures 2 and 3, respectively. After confirming the clones, glycerol stocks called Working Lot were made. For this purpose, were inoculated 10 pL from the Mother Stock in 10 ml of LB medium with 1% of glucose and 50 pg/ml of kanamycin and incubated at 37 ° C under agitation of 200 rpm, until they reached Absorbance (600nm) of approximately 1 0. Several aliquots with 500 pL of the cultivation and 500 pL of 50% sterile glycerol were prepared .The aliquots (Working Lot) were stored at -80 ° C.

### Example 3 - Analysis of L-asparaginase expression in shake flasks

To analyze the expression of the L-asparaginase enzyme cultures were performed in triplicate for each recombinant bacteria (*E. coli* BL21 (DE3)/p T28a/asparaginase and *E. coli* BL21 (DE3)/pET26b/asparaginase) in shake flasks. Therefore, each pre-inoculum was prepared by inoculating 10 uL of the Working Lot of the recombinant bacteria *E. coli* BL21 (DE3)/pET28a/asparaginase and E. *coli* BL21 (DE3)/pET26b/asparaginase in 10 ml of LB medium with 1 % of glucose and 50 pg/ml of kanamycin. The pre-inoculum of each bacterium was incubated for 16 hours at 37 ° C and 200 rpm in 50 ml shake flasks. After 16 hours, the inoculum of each bacterium was prepared in 50 ml of LB medium with 1% of glucose and 50 pg/ml of kanamycin were inoculated with 1 ml of the pre-inoculum in 250 mL flasks. The cultures were incubated at 37 ° C and 200 rpm until they reached the exponential phase of growth (approximately 1,0 of 600nm Absorbance). At this point, the protein expression was induced by 0.55 mM IPTG (isopropyl-PD-thiogalactopyranoside) for 4 hours. At the end of the four hours of expression the cell growth of crops was assessed by absorbance 600nm (Abs600nm). Samples of 1 ml were taken from crops of *E. coli* BL21 (DE3)/pET28a/asparaginase and 10 ml of crops of *E. coli* BL21 (DE3)/pET26b/asparaginase to examine the L-asparaginase expression by enzyme activity. To evaluate the expression of the protein obtained from the *E. coli* BL21 (DE3)/pET28a/asparaginase the cell pellets obtained from 1 ml of samples taken from cultures were used after 4 hours of expression. The precipitates were resuspended by adding 1 ml of 0.02 M sodium phosphate buffer, pH 7.3, and then subjected to ultrasound for five cycles of 10 seconds with 30% amplitude in a sonicator. These samples were then used for analysis of enzyme activity. To evaluate the expression of the protein obtained from the *E. coli* BL21 (SO3)/pET26b/asparaginase were used 10 ml of the cell-free culture medium removed from the crops after 4 hours of expression. These samples of culture medium were concentrated 10 times and the medium was replaced by 0.02 M pH 7.3sodium phosphate buffer, using ultrafiltration units with a 10 kDa membrane (Amicon Ultra-15, illipore) and centrifugations at 4000g for 30 minutes. The samples were then used for analysis of enzyme activity. For the analysis of enzymatic activity, 50 uL of the sample were added to 50 pL of 5 g/L asparagine. This reaction was incubated at 37 ° C for 30 minutes. Immediately after this time, 40 ul of this reaction were added to 10 pL of 1.5 M TCA (trichloroacetic acid). To the resulting 50 pL were added 2 ml of a reactant compound of a mixture of sodium salicylate, sodium nitroprusside and disodium EDTA and the mixture is stirred. In the sequence is added 2 ml of a second reagent compound of sodium hypochlorite and sodium hydroxide (Tabacco et al. Clinical Chemistry. 25 (2), 336-337, 1979). This is because the ammonium ions in the presence of these reagents form a compound chromogen blue-green. The mixture is incubated at 37 ° C for 5 minutes. The sample is then subjected to reading absorbance at a wavelength of 600nm. From a standard curve, were determined the concentrations of ammonium ion released during the enzymatic reaction. One international unit of asparaginase (IU) was defined as the quantity of enzyme that is capable of releasing 1 µτηoI ammonia per minute at 37 ° C and pH 7.3. The results obtained in these experiments, both cell growth and the expression of L-asparaginase, can be seen in Table 1 and 2. The enzyme activity results are presented as IU/ml of culture medium.

**TABLE 1**

| Recombinant bacteria | Average deviation Concentration (g/L) | Standard Deviation |
|---|---|---|
| *E.coli* BL21(DE3)/pET28a/asparaginase | 0,4565 | 0,018 |
| *E.coli* BL21(DE3)/pET26b/asparaginase | 0,8986 | 0,011 |

**TABLE 2**

| Recombinant bacteria | UI/mL (average) | Standard Deviation |
|---|---|---|
| *E.coli* BL21(DE3)/pET28a/asparaginase | 7,630 | 0,844 |
| *E.coli* BL21(DE3)/pET26b/asparaginase | 0,189 | 0,038 |

The results from these experiments show that L-asparaginase gene from *Zymomonas mobilis* was expressed in both recombinant bacteria produced. The L-asparaginase has been secreted into the culture medium when the bacterium *E. coli* recombinant BL21 (DE3)/pET26b/asparaginase was used for the expression, while the other recombinant bacteria produced in this study (*E. Coli* BL21 (DE3)/pET28a/asparaginase) was used, there was expression of the protein in the cytoplasm.

### Example 4 - Production of L-asparaginase in Bioreactor

The L-asparaginase enzyme was produced in bioreactors. A bioreactor was used for the cultivation of recombinant bacteria *E.coli* BL21 (DE3)/pET28a/asparaginase and enzyme production in the cytoplasm of the bacterium, another bioreactor was used for the cultivation of recombinant *E*. *coli* BL21 (DE3)/pET26b/asparaginase and enzyme production in the culture medium. Two pre-inoculums were made, one with *E. coli* BL21 (DE3)/pET28a/asparaginase and the other with *E. coli* BL21 (DE3)/pET26b/asparaginase. To this end, 10 ul of the bacteria working lot were inoculated into 50 ml of LB medium with 1% glucose and 50 pg/ml kanamycin in a 250 ml flasks. The pre-inoculum of each bacteria was incubated for 16 hours at 37 ° C with agitation of 200 rpm. After this time were used 8 ml of the pre-inoculum to inoculate a bioreactor containing 400ml of LB medium with 1% glucose and 50 pg/ml kanamycin. The cultures were conducted at 37 ° C under agitation of 200 rpm - 800 rpm. The pH of culture was maintained at 7.0. When the cell growth reached 600nm absorbance of approximately 2.0, the protein expression was induced by 0.55 mM IPTG for 4 hours. Samples were taken hourly to evaluate cell growth through 600nm absorbance measurements and after induction the samples were also taken to assess the expression of protein by enzyme activity analysis. The enzyme activity analyzes were conducted as described above in the analysis of samples taken from experiments in shake flasks.

In the cultivation using *E. coli* strain BL21 (DE3)/pET26b/asparaginase with extracellular L-asparaginase production was obtained the activity of 0.132 IU/ml culture medium and 172.7 IU/g cell after 4 hours of induction expression. The results of cell growth and protein production of this culture are shown in Figure 4. In the cultivation using *E. coli* strain BL21 (DE3)/pET28a/asparaginase with production of L-asparaginase was obtained by intracellular activity of 3.57 IU/ml of culture medium or 5185 IU/g cell after 4 hours of induction of expression. The results of cell growth and protein production of this cultivation are shown in Figure 5. It is also presented the values of specific activity of each crop (IU/mg total protein), the extracellular and intracellular L-asparaginase in Figure 6.

It is noteworthy that the production amounts of L-asparaginase presented in this patent application are higher than those presented by the native micro-organism of this L-asparaginase (*Zymomonas mobilis*), according to the culture in liquid medium in shake flasks, described in the brazilian patent application PI 0404952-7, which has the same applicant of the present one and in which activity was obtained 37.79 IU/g of cells in 33 hours of cultivation. It is clear that after our intervention presented in this patent application, it was possible to obtain approximately 135 times more IU/g cell, at a time four times lower.

### Example 5 - Cytotoxicity analysis of L-asparaginase

In those experiments we used the *Z. mobilis* recombinant L-asparaginase enzyme produced by recombinant bacterium *E*. *coli* BL21 (DE3)/pET26b/asparaginase described in this patent application. To verify the in vitro cytotoxicity of the L-asparaginase on the leukemic cells of a patient with acute lymphoblastic leukemia, it was used a primary sample of bone marrow obtained at diagnosis. Mononuclear cells, from human bone marrow sample from a patient 4 years old with acute lymphoblastic leukemia, were obtained at the time of their diagnosis, isolated by density gradient Ficoll-Paque. For the treatment with recombinant L-asparaginase, the cells were washed with RPMI 1640 culture medium, centrifuged and resuspended in L-glutamine-free RPMI 1640 culture medium, supplemented with 20% fetal bovine serum, 2 mmol/LL glutamine , 100 IU/ml penicillin and 100 vglmL of streptomycin. The cells were inoculated into tissue culture dishes of 24 wells, 10⁶ cells/well in 1 ml of medium with increasing concentrations of the recombinant L-asparaginase. In the control, the enzyme was not added. Both were incubated at 37 ° C in 5% CO2 humid atmosphere for 48 hours. After this period, cell viability was done by flow cytometry analysis. To assess viability, cells were marked with propidium iodide (PI). Before the acquisition, a compound pattern of two types of fluorescent microspheres was added to the samples. The data acquisition was done using the FACSDIVA software on a FACSCanto II flow cytometer (Becton Dickinson, Sans Jose, CA, USA). The data analysis was performed using the Infinicyte program (Cytognos SL, Salamanca, Spain).

The effect of the different concentrations tested on cell viability is shown by the marking of fluorophore propidium iodide (PI) in Figure 7, wherein the dead cells are marked with PI and emit fluorescence. The number of events and the percentage of death are shown in Table 3. It was observed that the percentage of dead cells in the samples that received treatment was higher than the control for all tested concentrations of enzyme, reaching more than 80% of death in concentration 1.0 IU/ml, however reaching approximately 79% at a concentration of 0.1 IU/ml.

**TABLE 3**

| Concentration (UI/mL) | Number of events | Number of dead cells | %Microspheres | % dead cells |
|---|---|---|---|---|
| 1,0 | 140.608 | 100.769 | 12.18 | 80.78 |
| 0,5 | 189.904 | 107.939 | 12.56 | 79.42 |
| 0,1 | 143.780 | 98.944 | 11.53 | 78.94 |
| 0,05 | 133.302 | 73.271 | 15.46 | 68.71 |
| 0,025 | 113.126 | 74.911 | 16.95 | 75.33 |
| 0,00* | 129.324 | 38.851 | 16.01 | 30.04 |

| | | | | |
|---|---|---|---|---|
| *Control | | | | |

The description made until this point of the L-asparaginases production process by means of synthetic gene construction and its insertion in *Escherichia coli,* object of the present invention, should be regarded only as a possible or possible embodiments, and any particular characteristics therein introduced shall be construed as illustrative, aiming only to facilitate understanding. Thus, they can in no way be considered as limitation to the invention, which is limited to the scope of the claims that follow.

### SEQUENCE LISTING

**SEQ ID NO: 1.**
   ➢ **optimized L-asparaginase** 
atgggccacc atcatcatca ccactccagc ggtcatatcg acgatgatga caaaatgaac

aatcaggttc actctatcca aactctgccg cgtatcctgg tgctggcaac cggcggtacc

atctctggca agaagaacgg catgtccgaa attggttata acgcaggcgg tgttactggc

aagcagctgg tagaagacat tccagaactg gcgaaactgg cagaaatcaa cgtagagcag

atcgcgaaca ttggtagcca ggacatgaac gatgcaatct ggctgcgcct ggcgaagcgt

atccaggatg cggtagcgca caatgaagca gatggcatcg tgatcaccca cggtaccgat

actatggaag aaaccgcatt cttcctggac accgttattc gtaccgataa accgatcatc

ctgaccggtg caatgcgtcc gtccaccgca atcggtgccg acggtccggc taacctgtac

gaagcaatcg aagtggcagc gaccccgaaa gcaaaggacc acggtgtaat gattgttatg

aacgatacca ttcatgcggc gcgttgggca tctaagaccc acactactgc cgtagaaacc

ttccagtcta tcaacgctgg cccgatcggt tatgtagacc cggcatctgt tcgtttcatt

gaaccgaaga agcagccggt tccgtcctat ggtctgccga ccactgcccc gctgccggcg

gtggaaatcc tgtatgcaca ctctggcatg ggcgcttcca tcatcaacga tctgattaaa

accggcgtaa aaggcatcat tctggcaggc gtgggtgatg gtaactcctc taaagaagca

atggctgcac tgaacctggc ggttaaacaa ggtgtgattg ttgtacgttc tagccgtacc

ggtagcggtt tcgtaaaccg taacgttgaa gtaaacgacg ataagaacga ttttgtggtg

agctatgacc tgagcccgca gaaggctcgc atcctgctgc aaatcctgat cgccaatggc

aagaacaagc tgagcgatat ccagagcgca ttcgaagcgg gtttctaa
**SEQ ID NO: 2** 
atgatgattt ttaaaatccc tgttaaggcc tcttctgctg cggccttggc aatatgcatg

atgatggggg ctactccggc gatatctatg aataatcagg ttcattcaat tcagacgtta

ccgcgcattt tagttctggc aacgggcggc acgatttccg gcaagaaaaa tggaatgtct

gaaatcggct ataatgcagg cggcgttact ggaaaacagc tcgttgaaga tataccggaa

ttagctaaac tcgctgaaat caatgtcgaa caaattgcca atatcggctc gcaagatatg

aatgatgcga tatggctgcg cttggccaag cgcatccaag acgccgtcgc ccataacgaa

gcggatggta ttgtgattac ccatggcacc gataccatgg aagaaaccgc ctttttcctt

gatacggtta ttcgcaccga caagccgatt attctgacag gcgccatgcg ccctagcact

gccattggtg cagatggtcc cgccaattta tatgaggcga ttgaagtcgc ggccaccccc

aaggccaaag atcatggcgt catgatcgtc atgaatgaca ctattcatgc agccagatgg

gcaagcaaaa cccacacaac cgccgtcgaa acctttcagt ccatcaatgc aggacctatc
 
ggttatgtcg atccggcttc ggtgcggttt attgagccga aaaaacagcc tgtcccaagc

tatggccttc cgacgactgc gcctttgcct gcggtcgaaa tcctttacgc ccatagcggt

atgggggctt caattatcaa tgatctcatc aaaacgggcg tgaaaggcat tattcttgcc

ggtgttggtg acgggaatag ttcaaaagaa gcgatggctg ccctcaatct tgccgtcaaa

caaggcgtga ttgttgtgcg ttcatccaga accggatcag gctttgtgaa tcgcaatgtc

gaggtcaatg atgacaaaaa cgactttgtt gtctcttatg atctttcgcc ccagaaagcc

cgcatccttc ttcagatttt aatagccaat ggcaaaaaca aactttctga tatccaatct

gcatttgaag ctggttttta
**SEQ ID NO: 3**
   **> optimized pET26b/asparaginase sequence** 
atccggatat agttcctcct ttcagcaaaa aacccctcaa gacccgttta gaggccccaa

ggggttatgc tagttattgc tcagcggtgg cagcagccaa ctcagcttcc tttcgggctt

tgttagcagc cggatctcag tggtggtggt ggtggtgctc gagttagaaa cccgcttcga

atgcgctctg gatatcgctc agcttgttct tgccattggc gatcaggatt tgcagcagga

tgcgagcctt ctgcgggctc aggtcatagc tcaccacaaa atcgttctta tcgtcgttta

cttcaacgtt acggtttacg aaaccgctac cggtacggct agaacgtaca acaatcacac

cttgtttaac cgccaggttc agtgcagcca ttgcttcttt agaggagtta ccatcaccca

cgcctgccag aatgatgcct tttacgccgg ttttaatcag atcgttgatg atggaagcgc

ccatgccaga gtgtgcatac aggatttcca ccgccggcag cggggcagtg gtcggcagac

cataggacgg aaccggctgc ttcttcggtt caatgaaacg aacagatgcc gggtctacat

aaccgatcgg gccagcgttg atagactgga aggtttctac ggcagtagtg tgggtcttag

atgcccaacg cgccgcatga atggtatcgt tcataacaat cattacaccg tggtcctttg

ctttcggggt cgctgccact tcgattgctt cgtacaggtt agccggaccg tcggcaccga

ttgcggtgga cggacgcatt gcaccggtca ggatgatcgg tttatcggta cgaataacgg

tgtccaggaa gaatgcggtt tcttccatag tatcggtacc gtgggtgatc acgatgccat

ctgcttcatt gtgcgctacc gcatcctgga tacgcttcgc caggcgcagc cagattgcat

cgttcatgtc ctggctacca atgttcgcga tctgctctac gttgatttct gccagtttcg

ccagttctgg aatgtcttct accagctgct tgccagtaac accgcctgcg ttataaccaa

tttcggacat gccgttcttc ttgccagaga tggtaccgcc ggttgccagc accaggatac
 
gcggcagagt ttggatagag tgaacctgat tgttcatttt gtcatcatcg tcgatatgac
cgctggagtg gtgatgatga tggtggccca tggccatcgc cggctgggca gcgaggagca
gcagaccagc agcagcggtc ggcagcaggt atttcatatg tatatctcct tcttaaagtt
aaacaaaatt atttctagag gggaattgtt atccgctcac aattccccta tagtgagtcg
tattaatttc gcgggatcga gatctcgatc ctctacgccg gacgcatcgt ggccggcatc
accggcgcca caggtgcggt tgctggcgcc tatatcgccg acatcaccga tggggaagat
cgggctcgcc acttcgggct catgagcgct tgtttcggcg tgggtatggt ggcaggcccc
gtggccgggg gactgttggg cgccatctcc ttgcatgcac cattccttgc ggcggcggtg
ctcaacggcc tcaacctact actgggctgc ttcctaatgc aggagtcgca taagggagag
cgtcgagatc ccggacacca tcgaatggcg caaaaccttt cgcggtatgg catgatagcg
cccggaagag agtcaattca gggtggtgaa tgtgaaacca gtaacgttat acgatgtcgc
agagtatgcc ggtgtctctt atcagaccgt ttcccgcgtg gtgaaccagg ccagccacgt
ttctgcgaaa acgcgggaaa aagtggaagc ggcgatggcg gagctgaatt acattcccaa
ccgcgtggca caacaactgg cgggcaaaca gtcgttgctg attggcgttg ccacctccag
tctggccctg cacgcgccgt cgcaaattgt cgcggcgatt aaatctcgcg ccgatcaact
gggtgccagc gtggtggtgt cgatggtaga acgaagcggc gtcgaagcct gtaaagcggc
ggtgcacaat cttctcgcgc aacgcgtcag tgggctgatc attaactatc cgctggatga
ccaggatgcc attgctgtgg aagctgcctg cactaatgtt ccggcgttat ttcttgatgt
ctctgaccag acacccatca acagtattat tttctcccat gaagacggta cgcgactggg
cgtggagcat ctggtcgcat tgggtcacca gcaaatcgcg ctgttagcgg gcccattaag
ttctgtctcg gcgcgtctgc gtctggctgg ctggcataaa tatctcactc gcaatcaaat
tcagccgata gcggaacggg aaggcgactg gagtgccatg tccggttttc aacaaaccat
gcaaatgctg aatgagggca tcgttcccac tgcgatgctg gttgccaacg atcagatggc
gctgggcgca atgcgcgcca ttaccgagtc cgggctgcgc gttggtgcgg atatctcggt
agtgggatac gacgataccg aagacagctc atgttatatc ccgccgttaa ccaccatcaa
acaggatttt cgcctgctgg ggcaaaccag cgtggaccgc ttgctgcaac tctctcaggg
ccaggcggtg aagggcaatc agctgttgcc cgtctcactg gtgaaaagaa aaaccaccct
ggcgcccaat acgcaaaccg cctctccccg cgcgttggcc gattcattaa tgcagctggc
acgacaggtt tcccgactgg aaagcgggca gtgagcgcaa cgcaattaat gtaagttagc
tcactcatta ggcaccggga tctcgaccga tgcccttgag agccttcaac ccagtcagct
ccttccggtg ggcgcggggc atgactatcg tcgccgcact tatgactgtc ttctttatca
 
tgcaactcgt aggacaggtg ccggcagcgc tctgggtcat tttcggcgag gaccgctttc
gctggagcgc gacgatgatc ggcctgtcgc ttgcggtatt cggaatcttg cacgccctcg
ctcaagcctt cgtcactggt cccgccacca aacgtttcgg cgagaagcag gccattatcg
ccggcatggc ggccccacgg gtgcgcatga tcgtgctcct gtcgttgagg acccggctag
gctggcgggg ttgccttact ggttagcaga atgaatcacc gatacgcgag cgaacgtgaa
gcgactgctg ctgcaaaacg tctgcgacct gagcaacaac atgaatggtc ttcggtttcc
gtgtttcgta aagtctggaa acgcggaagt cagcgccctg caccattatg ttccggatct
gcatcgcagg atgctgctgg ctaccctgtg gaacacctac atctgtatta acgaagcgct
ggcattgacc ctgagtgatt tttctctggt cccgccgcat ccataccgcc agttgtttac
cctcacaacg ttccagtaac cgggcatgtt catcatcagt aacccgtatc gtgagcatcc
tctctcgttt catcggtatc attaccccca tgaacagaaa tcccccttac acggaggcat
cagtgaccaa acaggaaaaa accgccctta acatggcccg ctttatcaga agccagacat
taacgcttct ggagaaactc aacgagctgg acgcggatga acaggcagac atctgtgaat
cgcttcacga ccacgctgat gagctttacc gcagctgcct cgcgcgtttc ggtgatgacg
gtgaaaacct ctgacacatg cagctcccgg agacggtcac agcttgtctg taagcggatg
ccgggagcag acaagcccgt cagggcgcgt cagcgggtgt tggcgggtgt cggggcgcag
ccatgaccca gtcacgtagc gatagcggag tgtatactgg cttaactatg cggcatcaga
gcagattgta ctgagagtgc accatatatg cggtgtgaaa taccgcacag atgcgtaagg
agaaaatacc gcatcaggcg ctcttccgct tcctcgctca ctgactcgct gcgctcggtc
gttcggctgc ggcgagcggt atcagctcac tcaaaggcgg taatacggtt atccacagaa
tcaggggata acgcaggaaa gaacatgtga gcaaaaggcc agcaaaaggc caggaaccgt
aaaaaggccg cgttgctggc gtttttcca aggctccgcc cccctgacga gcatcacaaa
aatcgacgct caagtcagag gtggcgaaac ccgacaggac tataaagata ccaggcgttt
ccccctggaa gctccctcgt gcgctctcct gttccgaccc tgccgcttac cggatacctg
tccgcctttc tcccttcggg aagcgtggcg ctttctcata gctcacgctg taggtatctc
agttcggtgt aggtcgttcg ctccaagctg ggctgtgtgc acgaaccccc cgttcagccc
gaccgctgcg ccttatccgg taactatcgt cttgagtcca acccggtaag acacgactta
tcgccactgg cagcagccac tggtaacagg attagcagag cgaggtatgt aggcggtgct
acagagttct tgaagtggtg gcctaactac ggctacacta gaaggacagt atttggtatc
tgcgctctgc tgaagccagt taccttcgga aaaagagttg gtagctcttg atccggcaaa
caaaccaccg ctggtagcgg tggttttttt gtttgcaagc agcagattac gcgcagaaaa
 
aaaggatctc aagaagatcc tttgatcttt tctacggggt ctgacgctca gtggaacgââ
aactcacgtt aagggatttt ggtcatgaac aataaaactg tctgcttaca taaacagtaa
tacaaggggt gttatgagcc atattcaacg ggaaacgtct tgctctaggc cgcgattaaa
ttccaacatg gatgctgatt tatatgggta taaatgggct cgcgataatg tcgggcaatc
aggtgcgaca atctatcgat tgtatgggaa gcccgatgcg ccagagttgt ttctgaaaca
tggcaaaggt agcgttgcca atgatgttac agatgagatg gtcagactaa actggctgac
ggaatttatg cctcttccga ccatcaagca ttttatccgt actcctgatg atgcatggtt
actcaccact gcgatccccg ggaaaacagc attccaggta ttagaagaat atcctgattc
aggtgaaaat attgttgatg cgctggcagt gttcctgcgc cggttgcatt cgattcctgt
ttgtaattgt ccttttaaca gcgatcgcgt atttcgtctc gctcaggcgc aatcacgaat
gaataacggt ttggttgatg cgagtgattt tgatgacgag cgtaatggct ggcctgttga
acaagtctgg aaagaaatgc ataaactttt gccattctca ccggattcag tcgtcactca
tggtgatttc tcacttgata accttatttt tgacgagggg aaattaatag gttgtattga
tgttggacga gtcggaatcg cagaccgata ccaggatctt gccatcctat ggaactgcct
cggtgagttt tctccttcat tacagaaacg gctttttcaa aaatatggta ttgataatcc
tgatatgaat aaattgcagt ttcatttgat gctcgatgag tttttctaag aattaattca
tgagcggata catatttgaa tgtatttaga aaaataaaca aataggggtt ccgcgcacat
ttccccgaaa agtgccacct gaaattgtaa acgttaatat tttgttaaaa ttcgcgttaa
atttttgtta aatcagctca ttttttaacc aataggccga aatcggcaaa atcccttata
aatcaaaaga atagaccgag atagggttga gtgttgttcc agtttggaac aagagtccac
tattaaagaa cgtggactcc aacgtcaaag ggcgaaaaac cgtctatcag ggcgatggcc
cactacgtga accatcaccc taatcaagtt ttttggggtc gaggtgccgt aaagcactaa
atcggaaccc taaagggagc ccccgattta gagcttgacg gggaaagccg gcgaacgtgg
cgagaaagga agggaagaaa gcgaaaggag cgggcgctag ggcgctggca agtgtagcgg
tcacgctgcg cgtaaccacc acacccgccg cgcttaatgc gccgctacag ggcgcgtccc
attcgcca
**SEQ ID NO: 4.**
   **> optimized pET28a/asparaginase sequence** 
atccggatat agttcctcct ttcagcaaaa aacccctcaa gacccgttta gaggccccaa
ggggttatgc tagttattgc tcagcggtgg cagcagccaa ctcagcttcc tttcgggctt
tgttagcagc cggatctcag tggtggtggt ggtggtgctc gagttagaaa cccgcttcga
atgcgctctg gatatcgctc agcttgttct tgccattggc gatcaggatt tgcagcagga
tgcgagcctt ctgcgggctc aggtcatagc tcaccacaaa atcgttctta tcgtcgttta
cttcaacgtt acggtttacg aaaccgctac cggtacggct agaacgtaca acaatcacac
cttgtttaac cgccaggttc agtgcagcca ttgcttcttt agaggagtta ccatcaccca
cgcctgccag aatgatgcct tttacgccgg ttttaatcag atcgttgatg atggaagcgc
ccatgccaga gtgtgcatac aggatttcca ccgccggcag cggggcagtg gtcggcagac
cataggacgg aaccggctgc ttcttcggtt caatgaaacg aacagatgcc gggtctacat
aaccgatcgg gccagcgttg atagactgga aggtttctac ggcagtagtg tgggtcttag
atgcccaacg cgccgcatga atggtatcgt tcataacaat cattacaccg tggtcctttg
ctttcggggt cgctgccact tcgattgctt cgtacaggtt agccggaccg tcggcaccga
ttgcggtgga cggacgcatt gcaccggtca ggatgatcgg tttatcggta cgaataacgg
tgtccaggaa gaatgcggtt tcttccatag tatcggtacc gtgggtgatc acgatgccat
ctgcttcatt gtgcgctacc gcatcctgga tacgcttcgc caggcgcagc cagattgcat
cgttcatgtc ctggctacca atgttcgcga tctgctctac gttgatttct gccagtttcg
ccagttctgg aatgtcttct accagctgct tgccagtaac accgcctgcg ttataaccaa
tttcggacat gccgttcttc ttgccagaga tggtaccgcc ggttgccagc accaggatac
gcggcagagt ttggatagag tgaacctgat tgttcatttt gtcatcatcg tcgatatgac
cgctggagtg gtgatgatga tggtggccca tggtatatct ccttcttaaa gttaaacaaa
attatttcta gaggggaatt gttatccgct cacaattccc ctatagtgag tcgtattaat
ttcgcgggat cgagatctcg atcctctacg ccggacgcat cgtggccggc atcaccggcg
ccacaggtgc ggttgctggc gcctatatcg ccgacatcac cgatggggaa gatcgggctc
gccacttcgg gctcatgagc gcttgtttcg gcgtgggtat ggtggcaggc cccgtggccg
ggggactgtt gggcgccatc tccttgcatg caccattcct tgcggcggcg gtgctcaacg
gcctcaacct actactgggc tgcttcctaa tgcaggagtc gcataaggga gagcgtcgag
atcccggaca ccatcgaatg gcgcaaaacc tttcgcggta tggcatgata gcgcccggaa
gagagtcaat tcagggtggt gaatgtgaaa ccagtaacgt tatacgatgt cgcagagtat
gccggtgtct cttatcagac cgtttcccgc gtggtgaacc aggccagcca cgtttctgcg
aaaacgcggg aaaaagtgga agcggcgatg gcggagctga attacattcc caaccgcgtg
 
gcacaacaac tggcgggcaa acagtcgttg ctgattggcg ttgccacctc cagtctggcc
ctgcacgcgc cgtcgcaaat tgtcgcggcg attaaatctc gcgccgatca actgggtgcc
agcgtggtgg tgtcgatggt agaacgaagc ggcgtcgaag cctgtaaagc ggcggtgcac
aatcttctcg cgcaacgcgt cagtgggctg atcattaact atccgctgga tgaccaggat
gccattgctg tggaagctgc ctgcactaat gttccggcgt tatttcttga tgtctctgac
cagacaccca tcaacagtat tattttctcc catgaagacg gtacgcgact gggcgtggag
catctggtcg cattgggtca ccagcaaatc gcgctgttag cgggcccatt aagttctgtc
tcggcgcgtc tgcgtctggc tggctggcat aaatatctca ctcgcaatca aattcagccg
atagcggaac gggaaggcga ctggagtgcc atgtccggtt ttcaacaaac catgcaaatg
ctgaatgagg gcatcgttcc cactgcgatg ctggttgcca acgatcagat ggcgctgggc
gcaatgcgcg ccattaccga gtccgggctg cgcgttggtg cggatatctc ggtagtggga
tacgacgata ccgaagacag ctcatgttat atcccgccgt taaccaccat caaacaggat
tttcgcctgc tggggcaaac cagcgtggac cgcttgctgc aactctctca gggccaggcg
gtgaagggca atcagctgtt gcccgtctca ctggtgaaaa gaaaaaccac cctggcgccc
aatacgcaaa ccgcctctcc ccgcgcgttg gccgattcat taatgcagct ggcacgacag
gtttcccgac tggaaagcgg gcagtgagcg caacgcaatt aatgtaagtt agctcactca
ttaggcaccg ggatctcgac cgatgccctt gagagccttc aacccagtca gctccttccg
gtgggcgcgg ggcatgacta tcgtcgccgc acttatgact gtcttcttta tcatgcaact
cgtaggacag gtgccggcag cgctctgggt cattttcggc gaggaccgct ttcgctggag
cgcgacgatg atcggcctgt cgcttgcggt attcggaatc ttgcacgccc tcgctcaagc
cttcgtcact ggtcccgcca ccaaacgttt cggcgagaag caggccatta tcgccggcat
ggcggcccca cgggtgcgca tgatcgtgct cctgtcgttg aggacccggc taggctggcg
gggttgcctt actggttagc agaatgaatc accgatacgc gagcgaacgt gaagcgactg
ctgctgcaaa acgtctgcga cctgagcaac aacatgaatg gtcttcggtt tccgtgtttc
gtaaagtctg gaaacgcgga agtcagcgcc ctgcaccatt atgttccgga tctgcatcgc
aggatgctgc tggctaccct gtggaacacc tacatctgta ttaacgaagc gctggcattg
accctgagtg atttttctct ggtcccgccg catccatacc gccagttgtt taccctcaca
acgttccagt aaccgggcat gttcatcatc agtaacccgt atcgtgagca tcctctctcg
tttcatcggt atcattaccc ccatgaacag aaatccccct tacacggagg catcagtgac
caaacaggaa aaaaccgccc ttaacatggc ccgctttatc agaagccaga cattaacgct
tctggagaaa ctcaacgagc tggacgcgga tgaacaggca gacatctgtg aatcgcttca
 
cgaccacgct gatgagcttt accgcagctg cctcgcgcgt ttcggtgatg acggtgaaaa
cctctgacac atgcagctcc cggagacggt cacagcttgt ctgtaagcgg atgccgggag
cagacaagcc cgtcagggcg cgtcagcggg tgttggcggg tgtcggggcg cagccatgac
ccagtcacgt agcgatagcg gagtgtatac tggcttaact atgcggcatc agagcagatt
gtactgagag tgcaccatat atgcggtgtg aaataccgca cagatgcgta aggagaaaat
accgcatcag gcgctcttcc gcttcctcgc tcactgactc gctgcgctcg gtcgttcggc
tgcggcgagc ggtatcagct cactcaaagg cggtaatacg gttatccaca gaatcagggg
ataacgcagg aaagaacatg tgagcaaaag gccagcaaaa ggccaggaac cgtaaaaagg
ccgcgttgct ggcgtttttc cataggctcc gcccccctga cgagcatcac aaaaatcgac
gctcaagtca gaggtggcga aacccgacag gactataaag ataccaggcg tttccccctg
gaagctccct cgtgcgctct cctgttccga ccctgccgct taccggatac ctgtccgcct
ttctcccttc gggaagcgtg gcgctttctc atagctcacg ctgtaggtat ctcagttcgg
tgtaggtcgt tcgctccaag ctgggctgtg tgcacgaacc ccccgttcag cccgaccgct
gcgccttatc cggtaactat cgtcttgagt ccaacccggt aagacacgac ttatcgccac
tggcagcagc cactggtaac aggattagca gagcgaggta tgtaggcggt gctacagagt
tcttgaagtg gtggcctaac tacggctaca ctagaaggac agtatttggt atctgcgctc
tgctgaagcc agttaccttc ggaaaaagag ttggtagctc ttgatccggc aaacaaacca
ccgctggtag cggtggtttt tttgtttgca agcagcagat tacgcgcaga aaaaaaggat
ctcaagaaga tcctttgatc ttttctacgg ggtctgacgc tcagtggaac gaaaactcac
gttaagggat tttggtcatg aacaataaaa ctgtctgctt acataaacag taatacaagg
ggtgttatga gccatattca acgggaaacg tcttgctcta ggccgcgatt aaattccaac
atggatgctg atttatatgg gtataaatgg gctcgcgata atgtcgggca atcaggtgcg
acaatctatc gattgtatgg gaagcccgat gcgccagagt tgtttctgaa acatggcaaa
ggtagcgttg ccaatgatgt tacagatgag atggtcagac taaactggct gacggaattt
atgcctcttc cgaccatcaa gcattttatc cgtactcctg atgatgcatg gttactcacc
actgcgatcc ccgggaaaac agcattccag gtattagaag aatatcctga ttcaggtgaa
aatattgttg atgcgctggc agtgttcctg cgccggttgc attcgattcc tgtttgtaat
tgtcctttta acagcgatcg cgtatttcgt ctcgctcagg cgcaatcacg aatgaataac
ggtttggttg atgcgagtga ttttgatgac gagcgtaatg gctggcctgt tgaacaagtc
tggaaagaaa tgcataaact tttgccattc tcaccggatt cagtcgtcac tcatggtgat
ttctcacttg ataaccttat ttttgacgag gggaaattaa taggttgtat tgatgttgga
 
cgagtcggaa tcgcagaccg ataccaggat cttgccatcc tatggaactg cctcggtgag

ttttctcctt cattacagaa acggcttttt caaaaatatg gtattgataa tcctgatatg

aataaattgc agtttcattt gatgctcgat gagtttttct aagaattaat tcatgagcgg

atacatattt gaatgtattt agaaaaataa acaaataggg gttccgcgca catttccccg

aaaagtgcca cctgaaattg taaacgttaa tattttgtta aaattcgcgt taaatttttg

ttaaatcagc tcatttttta accaataggc cgaaatcggc aaaatccctt ataaatcaaa

agaatagacc gagatagggt tgagtgttgt tccagtttgg aacaagagtc cactattaaa

gaacgtggac tccaacgtca aagggcgaaa aaccgtctat cagggcgatg gcccactacg

tgaaccatca ccctaatcaa gttttttggg gtcgaggtgc cgtaaagcac taaatcggaa

ccctaaaggg agcccccgat ttagagcttg acggggaaag ccggcgaacg tggcgagaaa

ggaagggaag aaagcgaaag gagcgggcgc tagggcgctg gcaagtgtag cggtcacgct

gcgcgtaacc accacacccg ccgcgcttaa tgcgccgcta cagggcgcgt cccattcgcc

a
**SEQ ID NO: 5.**

Met Gly His His His His His His Ser Ser Gly His Ile Asp Asp Asp Asp Lys

Met Asn Asn Gln Val His Ile Gln Thr Ser Leu Pro Arg Ile Leu Ala Val Leu

Thr Gly Gly Thr Ile Ser Gly Lys Gly Lys Asn Met Ser Glu Ile Gly Tyr Asn

Ala Gly Gly Val Thr Gly Lys Gln Leu Val Glu Asp Ile Glu Pro Leu Lys Ala

Ala Leu Glu Ile Asn Val Glu Gln Ile Ala Asn Ile Gly Ser Gln Asp Met Asn

Asp Ala Ile Trp Leu Arg Leu Ala Lys Arg Ile Gln Asp Ala Val Ala Glu Ala

His The Asp Gly Ile Val Ile Thr His Gly Thr Asp Thr Met Glu Glu Thr Ala

Phe Phe Leu Asp Thr Val Ile Arg Thr Asp Lys Pro Ile Ile Leu Thr Gly Ala

Met Arg Pro Ser Thr Ala Ile Gly Ala Asp Gly Pro Ala Asn Leu Tyr Glu Ala

Glu Ile Ala Val Ala Thr Pro Lys Lys Asp Ala His Gly Val Met Ila Val Met

Asn Asp Thr Ile His Ala Ala Arg Trp Ala Ser Lys Thr His Thr Thr Ala Val

Glu Thr Phe Gln Ser Ile Asn Ala Gly Pro Ile Gly Tyr Pro Val Asp Ala Ser

Arg Val Phe Ile Glu Pro Lys Lys Gln Val Pro Pro Leu Gly Tyr Ser Thr Thr

Ala Pro Pro Pro Leu Ala Val Glu Ile Leu Ala Tyr Met His Ser Gly Ala Gly

Ser Ile Ile Asn Asp Ile Leu Lys Thr Gly Lys Gly Ile Ile Val Leu Ala Gly

Val Gly Asp Gly Asn Ser Ser Lys Glu Ala Met Ala Ala Leu Asn Leu Ala Val

Lys Gln Gly Val Ile Val Val Arg Ser Ser Arg Thr Gly Ser Gly Phe Val Arg

Asn Asn Val Glu Val Asn Asp Asp Lys Asn Asp Phe Val Val Ser Tyr Asp Leu

Ser Pro Gln Lys Ala Arg Ile Leu Leu Gln Ile Leu Ile Ala Asn Gly Lys Asn

Lys Leu Ser Asp Ile Gln Ser Ala Phe Glu Ala Gly Phe

## Claims

1. SYNTHETIC DEOXYRIBONUCLEIC ACIDS (DNAs), **characterized by** comprising the optimized nucleotide sequences that encode into L-asparaginases *Zymomonas.*

2. SYNTHETIC DEOXYRIBONUCLEIC ACIDS (DNAs) as in claim 1, **characterized by** encoding the L-asparaginases of *Zymomonas mobilis.*

3. SYNTHETIC DEOXYRIBONUCLEIC ACIDS (DNAs) as in claim 1, **characterized by** SEQ ID NO: 1.

4. EXPRESSION VECTORS IN BACTERIA **characterized by** SEQ D NO: 3, SEQ ID NO: 4.

5. BACTERIA AND RECOMBINANT YEAST **characterized by** being transformed with the DNAs specified in claim 1.

6. RECOMBINANT BACTERIA characterized for being *Escherictria coli* containing the DNAs specified in claim 1,

7. RECOMBINANT YEAST characterized for being Pichia pastoris containing the DNA specified in claim 1.

8. FUSION PROTEIN, **characterized by** SEQ D NO: 5 obtained from deoxyribonucleic acid according to claim 1, represented by SEQ ID NO 1.

9. PRODUCTION PROCESS OF L-ASPARAGINASES BY BACTERIA, **characterized by** comprising the construction of synthetic DNAs according to claim 1, its insertion in the bacteria *Escherichia coli,* so that it passes to produce L-asparaginases in high levels of expression and high productivity

10. PRODUCTION PROCESS OF L-ASPARAGINASES BY YEAST, **characterized by** comprising the construction of synthetic DNAs according to claim 1, its insertion into the Pichia pastoris yeast, so that it passes to produce L-asparaginases in high expression levels and high productivity.

11. PRODUCING PROCESS OF L-ASPARAGINASES BY GENETIC MODIFICATION OF BACTERIAS OR YEASTS, according to claim 1, **characterized by** cultivation conducted between 15 and 37 ° C under agitation from 200 to 800rpm, and its pH maintained between 5.0 and 7.0.

12. COMPOSITION **characterized by** comprising L-asparaginase obtained from processes according to claims 9 and 10, composed of L-asparaginase with or without excipients such as mannitol, sorbitol, sodium chloride, dextrose.

13. USE OF L-ASPARAGINASES for preparing the base formulations L-asparaginase used to treat cancer, tumors, diseases of cell proliferation, as well as other medical applications.

14. METHOD OF TREATING cancer using the composition according to claim 12.
